# EUROPEAN PATENT SPECIFICATION

(11) **EP 0 394 295 B1**
(45) Date of publication and mention of the grant of the patent: **26.01.1994**
(21) Application number: 88909790.3
(22) Date of filing: 16.11.1988
(51) Int. Cl.: A61M 5/24

(54) **A DETACHABLE HOUSING FOR A SYRINGE**
ABNEHMBARES SPRITZENGEHÄUSE
LOGEMENT AMOVIBLE POUR SERINGUE

(30) Priority: 17.11.1987 GB 8726846; 11.03.1988 GB 8805815
(43) Date of publication of application: 31.10.1990
(73) Proprietor: SELDOREN LIMITED, Lancashire BL9 0ST (GB)
(72) Inventor: HYMANSON, Victor, Manchester M25 7LL (GB)
(74) Representative: Quest, Barry
(86) International application number: GB8801003
(87) International publication number: WO8904680

(56) References cited:
- GB-A- 2 202 148
- US-A- 3 820 652
- US-A- 3 930 499
- US-A- 4 581 023

## Description

### TECHNICAL FIELD

This invention relates to syringes, and in particular to a detachable housing for a syringe for giving injections of an anaesthetic or other drug to humans preparatory to or during dental, surgical or other medical treatment.

### BACKGROUND ART

For such purposes it is customary to provide a disposable hypodermic needle which is secured to a syringe for use and is then detached from the syringe and discarded. The syringe comprises a housing to accept a cartridge containing the drug and a plunger mechanism to exert a force on a bung located at one end of the cartridge and thus force the drug out of the cartridge via the needle. A container in which the needle is supplied, and its cap, are also disposable, the needle being returned into the container and the cap being replaced on the container prior to such disposal. By this means it is intended to ensure that a patient is not contaminated due to the use of an other than unused needle. Such a needle has a body secured thereto by means of which the needle may be handled and attached to the syringe housing, the needle protruding from the body at opposed ends thereof. The distal end of the needle is, in use, inserted into the patient, whilst the proximal end is inserted into the syringe to penetrate the cartridge of anaesthetic or other drug contained in the syringe housing. During attachment of a needle to the syringe, or removal therefrom, it is possible that the proximal end of the needle can come into contact with the end of the syringe housing. In consequence, unless the syringe housing is sterilised after each use, there is a possibility that a new and unused needle can become contaminated on attachment to the syringe by deposits left thereon during withdrawal therefrom of a previously used needle. Also difficulty arises in replacing the needle in its container for disposal purposes, since the operator is liable to contact with or even be pierced by the exposed distal end of the needle during this operation.

US-A-3,930,499 (Rimbaud) discloses a syringe with parts which are disposable after use, comprising a head with an axially slidable plunger, the head being connectable to a disposable tubular barrel having an end with a boss through which passes a needle, so that an end of the needle projects some distance into the interior of the barrel. A cartridge or other dosage container can be fitted into the barrel, with a displaceable bung at one end to act as a piston member when pushed by the plunger, and a stopper at the other, which can be pierced by the inner end of the needle when the cartidge is fully pushed into the barrel. The specification teaches that a second (or further) injection may be performed with the same syringe by replacing the cartridge. In doing so the same needle must be reused, since as this is firmly embedded in the end boss of the barrel, it cannot be removed without risk of scratching or skin puncturing by the needle.

It is an object of the present invention to provide a means whereby such possibilities are avoided or the risks thereof can be substantially reduced.

### DISCLOSURE OF THE INVENTION

According to the invention there is provided a detachable housing for a syringe of the kind comprising a barrel containing a bore for retention of a drug therein, said barrel having a first attachment means at one end for attachment to a plunger mechanism and a second attachment means at the opposite end for attachment to a needle, a piston member being moveably located in the bore at or adjacent said one end of the barrel, a penetrable membrane being provided across the bore at or adjacent said opposite end of the barrel, and a drug being retained in the bore between the piston member and the penetrable membrane prior to penetration of said membrane characterised in that said bore is formed integrally with the barrel, whereby after use, the barrel can be detached from the plunger mechanism for disposal of the housing as a unit including with the needle, the barrel, the piston member and the penetrable membrane.

With this arrangement, it will be appreciated that, after use, the entire housing as well as the needle can be discarded and replaced by a fresh drug-containing housing with minimal handling of the housing and needle, and especially without requiring sterilisation of the housing. Preferably, therefore, the housing is formed from an inexpensive and conveniently disposable material such as a plastics material.

The housing barrel or at least a central portion thereof between the said ends defining the said bore may be a one-piece structure with the bore comprising an inner surface of the barrel. If desired, however, the housing barrel may be formed in one or more interconnected parts and/or may have an internal sleeve or lining or coating or the like to facilitate sealing and/or sliding of the piston member and this sleeve or lining or coating is bonded to or frictionally engaged with or otherwise fixed relative to the barrel.

With regard to the first attachment means this may comprise a screw thread, or other attachment arrangement.

With regard to the second attachment means this may comprise a screw thread or snap fit construction or any other suitable arrangement which may be such as to permit subsequent removal of the needle or to prevent such removal. It is also possible to utilise a penetrable member which also acts as the attachment means e.g. a relatively thick bung or the like which grips the needle when penetrated thereby.

With regard to the piston member this may extend entirely across the bore directly in tight contact with the bore (or the aforesaid sleeve/coating/lining) so as to provide a moveable seal, and thus the piston member may comprise a rubber or plastics bung or the like. Alternatively or additionally, the piston member may slidably engage a sealing element beneath the piston member.

The penetrable membrane may extend entirely across the bore (or the aforesaid sleeve/coating/lining) and may be fixed relative thereto. Such fixing may be achieved by bonding, or by frictional engagement or in any other suitable manner. The penetrable membrane may even be formed as an integral one-piece moulding with the barrel or at least with the pertaining end of the barrel. The penetrable membrane may be relatively thick (e.g. comparable to the thickness of the piston member) and thus for example may be of the nature of a rubber or plastics bung or the like, or it may comprise a thin membrane.

The drug may be retained in the bore such that it is directly in contact with the barrel.

In a preferred embodiment of the invention said barrel has a sleeve therearound which is slidable longitudinally thereof between a first operative position in which it overlies said barrel and a second disposal position in which it extends beyond said barrel in a direction away from said plunger mechanism to overlie a needle protruding from said barrel. This facilitates disposal of the needle with the housing after use with reduced risk of injury with the used needle.

Cooperating location means may be provided on said barrel and said sleeve to locate said sleeve in each of said positions. Said cooperating location means may comprise a ridge on one and a groove on the other of said sleeve and said barrel.

### BRIEF DESCRIPTION OF DRAWINGS

In order that the invention may be more readily understood specific embodiments thereof will now be described with reference to the drawings in which:
Fig. 1 is a sectional view of one embodiment of the first aspect of the invention;
Fig. 2 is a sectional view of one embodiment of the second aspect of the invention.

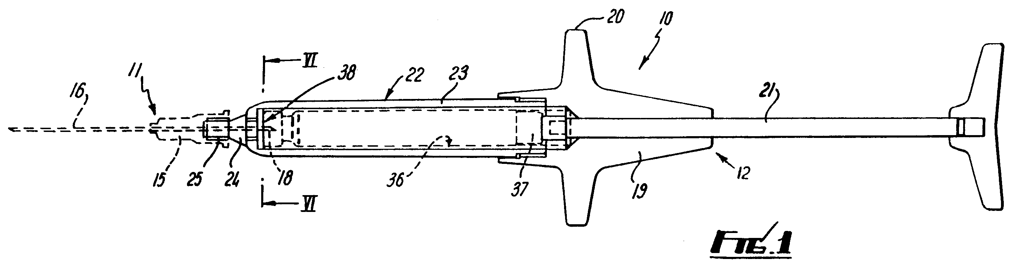

Referring now to Figure 1 there is shown a syringe 10 comprising a needle 11 and a plunger mechanism 12 and a housing 22. The needle 11 has a body 15 secured thereon for example a body of plastics material moulded therein. The plunger mechanism 12 has a body part 19 with a finger grip 20 and a plunger 21 . The housing 22 comprises a one-piece tubular, rigid barrel 23 of circular cross-section moulded from plastics material. One end of the barrel 23 is detachably connected to the body part 19 by a screw thread on the end of the barrel 23 which is screwed into a threaded bore in the end of the body part 19 of the plunger mechanism 12. The other end of the barrel 23 is provided with attachment means such that a housing cap 24 may be secured to the end of the barrel 23 for example by screwing thereto. The housing cap 24 has an end part 25 of reduced diameter to which the body 15 of the needle 11 can be attached.

The barrel 23 of the housing 22 defines an internal cylindrical bore 36. A rubber or plastics bung 37 is slidably located in the bore 36 towards the end of the barrel 23 which is connected to the body part 19. A penetrable membrane 38 manufactured from for example plastics material is provided across the other end of the bore 36 which is remote from the body part 19. The width of the penetrable membrane 38 is such that the penetrable membrane 38 may be readily pierced by the proximal end 18 of the needle 11. The membrane 38 may be fixed in position in any suitable manner. For example, it may be bonded in position or it may be moulded integrally with the barrel.

Piercing of the membrane 38 by the proximal end of the needle is achieved when the body 15 of the needle 11 is connected to the housing cap 25. On piercing thereof the penetrable membrane 38 acts to form a seal around the needle 11 such that the material to be injected may substantially only be allowed to pass out at the bore 36 via the needle 11. Connection of the needle body 15 to the cap 25 may be achieved in any suitable manner e.g. by a press fit or screwing or otherwise. Reference is made to GB-A-2 202 148 in this respect.

In use the housing 22 is supplied pre-filled with the material to be injected, such as an anaesthetic drug, located within the bore 36 and sealed therewithin by the membrane 38 and the bung 37. The barrel 23 is detachably connected to the body part 19 of the plunger mechanism 10 and the housing cap 25 is attached to the opposite end of the barrel 23. The body 15 of the needle 11 is fixed to the housing cap 25 such that the proximal end 18 of the needle penetrates the penetrable membrane 38. The drug to be injected may then be forced out of the bore via the needle by pushing the bung 37 with the plunger 21 towards the needle 11. Once the drug has been injected the barrel (23) may be detached from the plunger mechanism 21 and the housing 22, i.e. the barrel (23), the needle (11), the piston member (37) and the penetrable membrane (38) may then be disposed of.

As described above the drug is contained in the housing 22 in direct contact with the surface of the bore in the barrel 23.

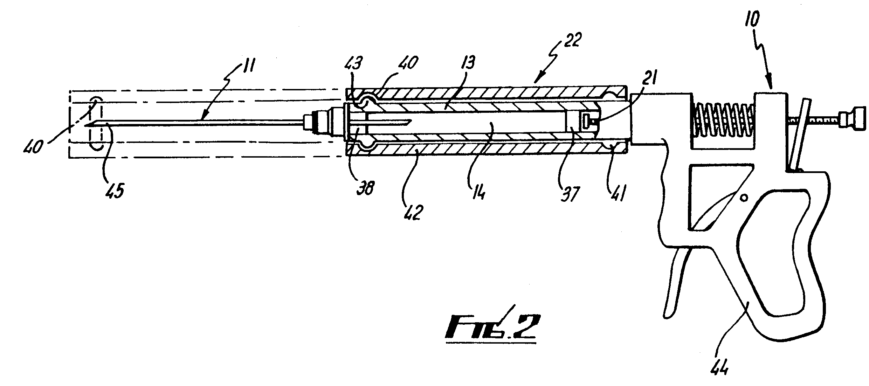

Referring now to Figure 2 there is shown one embodiment of the second aspect of the invention.

A syringe comprises a plunger mechanism 10 with a handle 44 and a housing 22 with a one-piece rigid moulded plastics barrel 13. The barrel 13 contains a drug 14 to be injected and is attached at one end to the mechanism 10, for example by screwing thereon, e.g. in similar manner to connection of the barrel in the embodiment of Fig. 1. The end of the barrel which is attached to the mechanism 10 has a piston member 37 in the form of a slidable plug therein which can be moved by a plunger 21 of the mechanism 10. The other end of the barrel 13 is closed by a penetrable membrane 38 for example in the form of a plug which is pierced by the proximal end of a needle 17 when the latter is attached to the housing 22. The piston member 37 and the membrane 38 fit within and seal a bore defined within the barrel 13.

Around the barrel 13 is a sleeve 42, which initially is retained in the operative position shown by means of a ridge 43 on the barrel 13 engaging in a groove 40 provided inside the sleeve 42. When the injection has been completed the sleeve 42 is slid longitudinally of the barrel 13 until a second groove 41 provided at the other end of the sleeve 42 engages the ridge 43 on the barrel 13. The sleeve 42 is then in the disposal position shown in dashed lines in the figure, and extends beyond the barrel 13 away from the injection device 10 so as to substantially enclose the distal end 45 of the needle 11.

By this means the needle 11 can be safely disposed of, with the spent housing 22, without having to replace the needle 11 in its original container. In consequence the risks associated with such replacement are avoided. The spent housing 22 is replaced with a fresh housing supplied pre-filled with the drug whereby sterilisation of the housing, or of associated parts used to mount the needle, is not required.

Other embodiments in accordance with the invention will be readily apparent to persons skilled in the art. For example, with the embodiment of Fig. 2, the annular ridge 43 on the barrel 13 and grooves 40, 41 in the sleeve 42 may be interchanged or may be replaced by other location means such as single or multiple individual bosses and recesses.

## Claims

1. A detachable housing (22) for a syringe of the kind comprising a barrel (23 or 13) containing a bore (36) for retention of a drug therein, said barrel (23) having a first attachment means at one end for attachment to a plunger mechanism (12) and a second attachment means at the opposite end for attachment to a needle (11), a piston member (37) being moveably located in the bore (36) at or adjacent said one end of the barrel (23), a penetrable membrane (38) being provided across the bore (36) at or adjacent said opposite end of the barrel, and a drug being retained in the bore (36) between the piston member (37) and the penetrable membrane (38) prior to penetration of said membrane (38) characterised in that said bore (36) is formed integrally with the barrel (23), whereby after use, the barrel (23) can be detached from the plunger mechanism (21) for disposal of the housing (22) as a unit including with the needle (11), the barrel (23), the piston member (37) and the penetrable membrane (38).

2. A detachable housing according to claim 1 characterised in that said barrel (13) has a sleeve (42) therearound which is slidable longitudinally thereof between a first operative position in which it overlies said barrel (13) and a second disposal position in which it extends beyond said barrel in a direction away from said plunger mechanism (12) to overlie a needle (11), protruding from said barrel.

3. A detachable housing according to claim 2 characterised in that cooperating location means (43, 40, 41) are provided on said barrel (13) and said sleeve (42) to locate the sleeve in each of said positions.

4. A detachable housing according to claim 3 characterised in that said cooperating location means comprise a ridge (43) on one and a groove (40, 41) on the other of said sleeve (42) and said barrel (13).

5. A detachable housing according to claim 1 characterised in that the barrel (23 or 13) is a one-piece structure.

6. A detachable housing according to any one of claims 1 to 5 characterised in that the piston member (37) comprises a moveable bung which seals said bore (36).

7. A detachable housing according to any one of claims 1 to 6 characterised in that the penetrable membrane (38) extends across and seals the bore (36).

8. A detachable housing according to any one of claims 1 to 7 characterised in that the drug is in direct contact with the surface of the bore (36).

## Patentansprüche

1. Abnehmbares Gehäuse (22) für eine Spritze mit einem Gefäß (23 oder 13), das mit einer Bohrung (36) zur Aufnahme eines Arzneimittels versehen ist; das Gefäß (23) weist an einem Ende eine erste Befestigungsvorrichtung zur Befestigung an einem Plunger (12) und am entgegengesetzten Ende eine zweite Befestigungsvorrichtung zur Befestigung an einer Nadel (11) auf, wobei an oder nahe dem einen Ende des Gefäßes (23) ein Kolben (37) in der Bohrung (36) bewegbar angeordnet ist und an oder nahe dem entgegengesetzten Ende des Gefäßes eine durchlässige Membrane (38) die Bohrung (36) abdeckt und zwischen dem Kolben (37) und der durchlässigen Membrane (38) vor dem Durchstoßen dieser Membrane (38) ein Arzneimittel in der Bohrung (36) enthalten ist, dadurch gekennzeichnet, daß die Bohrung (36) mit dem Gefäß (23) einstückig ausgebildet ist, so daß das Gefäß (23) nach Gebrauch von dem Plunger (21) zu lösen und das Gehäuse (22) als Ganzes einschließlich der Nadel (11), des Gefäßes (23), des Kolbens (37) und der durchlässigen Membrane (38) zu entsorgen ist.

2. Abnehmbares Gehäuse nach Anspruch 1,
dadurch gekennzeichnet, daß das Gefäß (13) von einer Hülse (42) umgeben ist, die in Längsrichtung des Gefäßes zwischen einer ersten Arbeitsposition, in der sie das Gefäß (13) umgibt, und einer zweiten Entsorgungsposition verschiebbar ist, in der sie über das Gefäß in einer von dem Plunger (12) abgewandten Richtung hinausragt und eine Nadel (11) umgibt, die aus dem Gefäß herausragt.

3. Abnehmbares Gehäuse nach Anspruch 2,
dadurch gekennzeichnet, daß an dem Gefäß (13) und der Hülse (42) Positioniervorrichtungen (43, 40, 41) vorgesehen sind, die miteinander zusammenwirken und die Hülse in der jeweiligen Lage positionieren.

4. Abnehmbares Gehäuse nach Anspruch 3,
dadurch gekennzeichnet, daß die miteinander zusammenwirkenden Positioniervorrichtungen eine Rippe (43) und eine Nut (40, 41) an dem Gefäß (13) bzw. der Hülse (42) aufweisen.

5. Abnehmbares Gehäuse nach Anspruch 1,
dadurch gekennzeichnet, daß das Gefäß (23 oder 13) einstückig ausgebildet ist.

6. Abnehmbares Gehäuse nach einem der Ansprüche 1 bis 5,
dadurch gekennzeichnet, daß der Kolben (37) einen bewegbaren Zapfen aufweist, der die Bohrung (36) dicht abschließt.

7. Abnehmbares Gehäuse nach einem der Ansprüche 1 bis 6,
dadurch gekennzeichnet, daß die durchlässige Membrane (38) die Bohrung (36) abdeckt und abdichtet.

8. Abnehmbares Gehäuse nach einem der Ansprüche 1 bis 7,
dadurch gekennzeichnet, daß das Arzneimittel in unmittelbarem Kontakt mit der Oberfläche der Bohrung (36) steht.

## Revendications

1. Boîtier amovible (22) pour seringue, du type comportent un tube (23 ou 13) comportant un alésage (36) pour retenir un médicament dans celui-ci, ledit tube (23) ayant des premiers moyens de fixation situés à une extrémité pour fixer un mécanisme à piston (12) et des seconds moyens de fixation à l'extrémité opposée pour fixer une aiguille (11), un élément formant piston (37) situé de manière à pouvoir se déplacer dans l'alésage (36) à ou à proximité de ladite extrémité du tube (23), et une membrane pouvant être percée (38) étant prévue en travers de l'alésage (36) à ou à proximité de ladite extrémité opposée du tube, et un médicament étant retenu dans l'alésage (36) entre l'élément formant piston (37) et la membrane pouvant être percée (38) avant le percement de ladite membrane (38) caractérisé en ce que ledit alésage (36) est venu de matière avec la tube (23), de sorte qu'après utilisation, le tube (23) peut être séparé du mécanisme à piston (21) pour jeter le boîtier (22) sous forme d'un ensemble compremant l'aiguille (11), le tube (23), l'élément formant piston (37) et la membrane pouvant être percée (38).

2. Boîtier amovible selon la revendication 1, caractérisé en ce que ledit tube (13) comporte un manchon (42) entourant celui-ci qui peut coulisser de manière longitudinale entre une première position active dans laquelle il entoure ledit tube (13) et une seconde position de mise au rebut dans laquelle il s'étend au-delà dudit tube dans la direction s'éloignant du mécanisme à piston (12) pour entourer l'aiguille (11), faisant saillie à partir dudit tube.

3. Boîtier amovible selon la revendication 2, caractérisé en ce que des moyens de positionnement coopérants (43, 40, 41) dont prévus sur ledit tube (13) et ledit manchon (42) pour positionner le manchon dans chacune desdites positions.

4. Boîtier amovible selon la revendication 3, caractérisé en ce que lesdits moyens de positionnement coopérants comportent une nervure (43) située sur l'un dudit manchon (42) et dudit tube (13) et une gorge (40, 41) située sur l'autre.

5. Boîtier amovible selon la revendication 1, caractérisé en ce que le tube (23 ou 13) est une structure en une seule pièce.

6. Boîtier amovible selon l'une quelconque des revendications 1 à 5, caractérisé en ce que l'élément formant piston (37) comporte un tampon pouvant être déplacé qui assure l'étanchéité dudit alésage (36).

7. Boîtier amovible selon l'une quelconque des revendications 1 à 6, caractérisé en ce que la membrane pouvant être percée (38) s'étend en travers de l'alésage (36) et assure l'étanchéité de ce dernier.

8. Boîtier amovible selon l'une quelconque des revendications 1 à 7, caractérisé en ce que le médicament est en contact direct avec la surface de l'alésage (36).
